(19) Europäisches Patentamt
European Patent Office
Office européen des brevets

(11) Publication number: 0 300 800 B1

(12) EUROPEAN PATENT SPECIFICATION

(45) Date of publication of patent specification: 16.10.91 Bulletin 91/42

(51) Int. Cl.$^5$: C07C 69/33, C07C 69/92, G02F 1/13

(21) Application number: 88306722.5

(22) Date of filing: 21.07.88

# (54) Macrocyclic tetramers having columnar tridimensional mesophases.

(30) Priority: 21.07.87 IT 2137087

(43) Date of publication of application: 25.01.89 Bulletin 89/04

(45) Publication of the grant of the patent: 16.10.91 Bulletin 91/42

(84) Designated Contracting States: CH DE FR GB IT LI NL

(56) References cited:
US-A- 4 619 788
CHEMICAL ABSTRACTS, vol. 93, no. 21, November 24, 1980, Columbus, Ohio, USA; A.G.S. HOEGBERG: "Two stereoisomeric macrocyclic resorcinol-acetaldehyde condensation products", page 595, column 1, abstract no. 203 571e

(73) Proprietor: Montedison S.p.A.
31, Foro Buonaparte
I-20121 Milan (IT)

(72) Inventor: Dalcanale, Enrico
14, via Visentin
I-28100 Novara (IT)
Inventor: Bonsignore, Stefanio
78, via Papa Giovanni XXIII
I-28100 Novara (IT)
Inventor: Du Vosel, Annick
15, via Ballario
I-28100 Novara (IT)

(74) Representative: Lamb, John Baxter et al
MARKS & CLERK 57-60 Lincoln's Inn Fields
London WC2A 3LS (GB)

EP 0 300 800 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to macrocyclic tetramers, which form stable, columnar, tridimensional mesophases. By the term "columnar tridimensional mesophases", as used in the present specification and claims, there is meant a structure ordered in three dimensions, wherein the molecules arrange themselves as columns. This type of mesophase is described, e.g., in "Journal de Physique", 1985, 47, page 351.

U.S. Patent No. 4,619,788 discloses hexa-substituted derivatives of tribenzo-cyclo-nona-triene having a mesophase of the pyramidal type. These are molecules provided with a central rigid core having a pyramidal structure, wherein the substituent chains are symmetrically bonded to the base of the pyramid. These compounds have an electrical dipolar moment which makes them particularly suitable for use as "memory devices" or as displays for opto-electronic devices.

It has now been found, in accordance with the present invention, that octa-substituted derivatives of the macrocyclic tetramer obtained by condensing resorcinol with an alkyl aldehyde, wherein the substituents are alkanoyl, p-alkylbenzoyl or p-alkoxybenzoyl chains, are products which form stable, columnar, tridimensional mesophases.

According to the invention therefore, there are provided macrocyclic tetramers, having stable columnar tridimensional mesophases, of the formula :

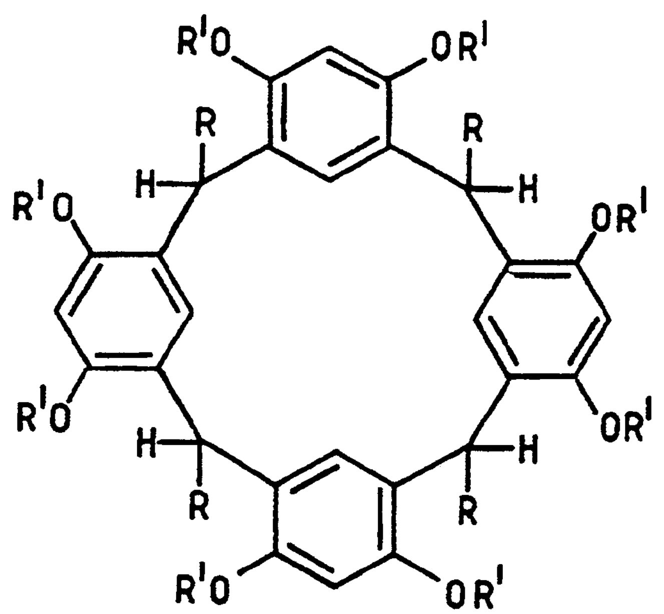

(I)

wherein

R is a $C_1$-$C_3$ alkyl group ; and the groups R' are the same or are different and each is a group of the formula:

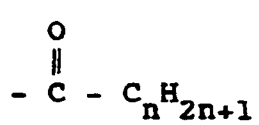

(II)

(in which n is an integer of 10 or more),

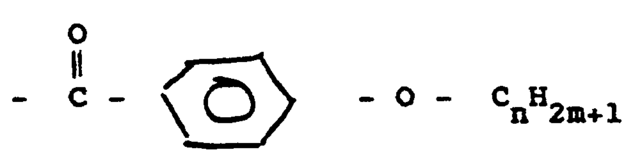

(III)

(in which m is an integer of 11 or more), or

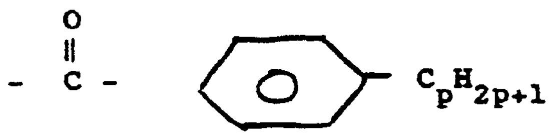

(IV)

(in which p is an integer of 11 or more).

Preferred tetramers of the formula (I) are those wherein n is an integer of from 11 to 25, and m and p are integers of from 11 to 25.

The R' substituents of the same molecule may be the same as or different from one another ; however, those compounds wherein the R' groups are the same are preferred.

In the following description reference will be made to the accompanying drawings in which :

Fig. 1 is a perspective view of a molecule of a tetramer of the invention ; and

Fig. 2 shows phase transition temperatures for various tetramers of the invention.

In the macrocyclic tetramers of the invention, the R groups are positioned in an axial position, as shown in Figure 1, which represents a perspective view of the molecule. This type of structure tends to form stable, tridimensional, columnar type mesophases wherein the crystalline lattice is formed with the central macrocyclic rings stacked one on the other in ordered columns, each ring being surrounded with alkyl chains in the melted state.

The tetramers of the formula (I) have melting temperatures of from 0° to 150°C, and the tridimensional mesophase arrangement may be evidenced by analysis on a differential scanning calorimeter (DSC), analysis under an optical microscope under polarized light, or X-ray diffraction.

The temperatures at which the molten material turns into isotropic material (the clearing points) are generally within the range of from 20° to 250°C.

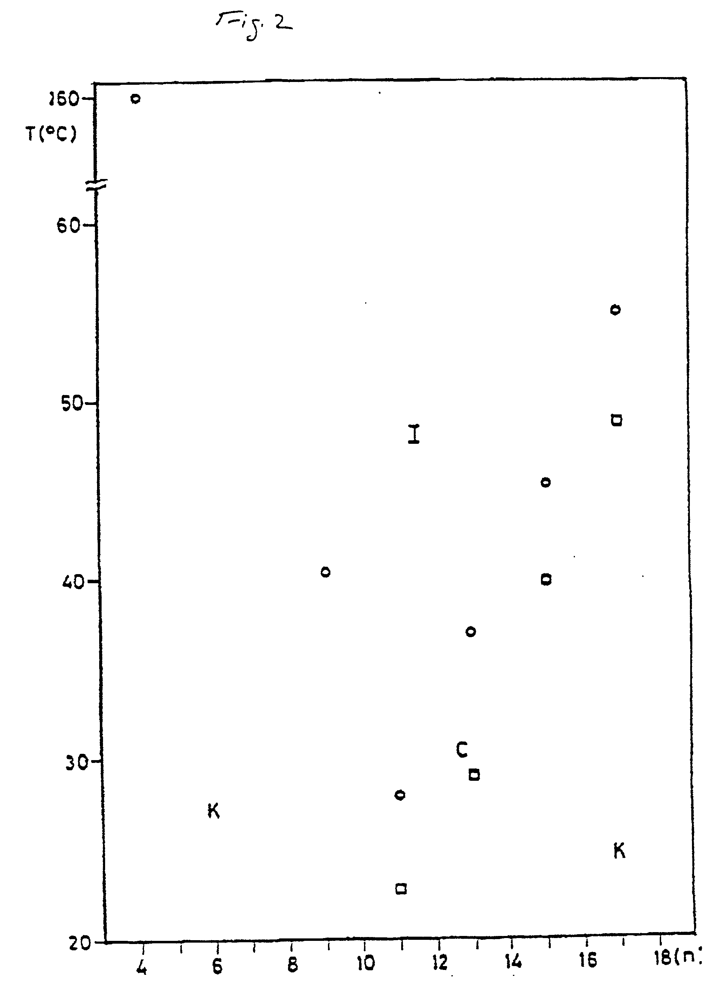

In Figure 2, there are shown, by way of example, transition temperatures between various phases for macrocyclic tetramers of formula (I), wherein R is $—CH_3$ and R' is the radical :

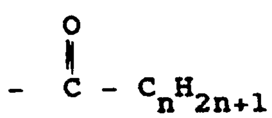

In Figure 2, the temperatures are shown on the ordinate while on the abcissa are shown the values of n. The letters I, C and K, respectively indicate the isotropic phase, the columnar tridimensional mesphase and the crystalline phase.

In particular, in Figure 2 the points marked by small cicles represent clearing points, and those marked by small squares represent the crystal-columnar mesophase transition.

The macrocyclic tetramers of the invention show a dipolar moment different from zero, which makes them suitable for use in the opto-electronics field, or, more particularly, in the field of photonics as components for non-linear optical devices. Moreover, the tetramers may be ferro-electric when the columns have the same direction of polarization and, therefore, they may be used as memory devices.

The macrocyclic tetramers of the invention may be prepared by reacting a macrocyclic tetramer of the formula :

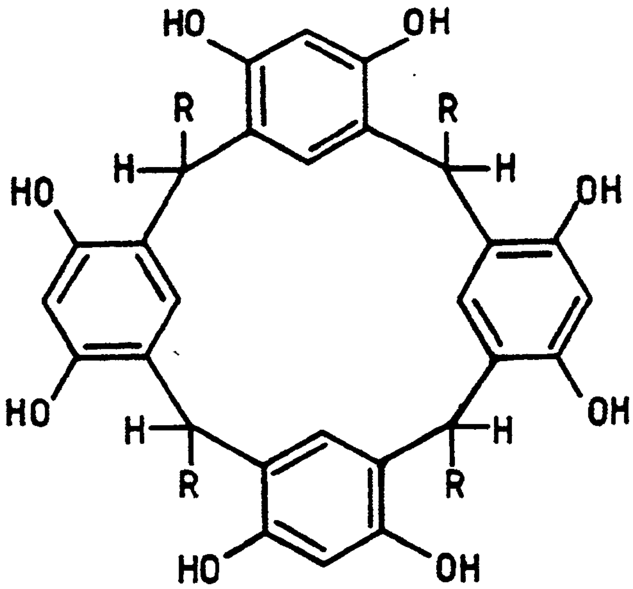

(V)

(in which R has the meaning defined above) with an alkanoyl halide of the formula R'-X (wherein X is a halogen atom, preferably chlorine ; and R' has the meaning defined above). Examples of suitable alkanoyl halides are lauroyl chloride, myristoyl chloride, palmitoyl chloride, stearoyl chloride, para-pentylbenzoyl chloride, p-dodecyloxybenzoyl chloride, p-hexylbenzoyl chloride, p-hexadecanoyloxybenzoyl chloride, and so forth.

The reaction may be carried out as a normal solventless esterification under ambient pressure and at a temperature of from 50° to 200°C.

Alternatively, the reaction may be carried out in a solvent vehicle of basic character, such as pyridine or a tertiary amine. In this case, the reaction temperature is preferably from 0° to 50°C.

The cyclic tetramers of formula (V) are per se known products, obtained by condensing resorcinol with alkyl aldehydes according to and as disclosed in "Journal of the American Chemical Society", 1932, 54, page 4325, and in "Journal of Organic Chemistry", 1980, 45, page 4498. According to these processes, the products of formula (V) may be obtained as two configurational isomers, respectively demoninated the "boat isomer" and the "chair isomer", and separable by means of known techniques, e.g. by crystallization.

Only those compounds which are obtained by starting from the boat isomer, with all R groups thereof being in the axial position, form stable, tridimensional, columnar type mesophases.

In order that the invention may be well understood, the following Examples are given by way of illustration only.

Example 1

Preparation of 3, 5, 10, 12, 17, 19, 24, 26 — octadecanoyloxy-r-1, c-8, c-15, c-22-tetramethyl-[14]-metacyclophane.

0.545 g (1 mM) of the tetramer of formula (V) in which R is $CH_3$ and 17.7 ml of palmitoyl chloride were heated for 6 hours at approximately 180°C with stirring. After reaction the excess chloride was distilled off under vacuum (120°C/ 5 x $10^{-2}$ mm, Hg). The remaining residue was dissolved in $CH_2Cl_2$ and the resulting solution was first mixed and stirred with 0.1 N NaOH, then washed $H_2O$ to netral pH, and finally thoroughly dried over sodium sulphate. The solvent was evaporated off, and the raw product was run twice through a silica gel column with 8/2 $CH_2Cl_2$/toluene and 8/2 $CH_2Cl_2$/hexane as eluents.

1.44 g of pure product was obtained (yield 59%).

Mass ($DCl^+$) ; $MH^+$ = 2449

NMR ($CDCl_3$) : 0.89 ppm [t, 24H, J=6, 8HZ,

$(CH_2)_n$-$CH_3$] ;

1.35 [bs, 192H, $(CH_2)_{12}$] ;

1.45 (D, 12H, J=7, 4Hz, CH-$CH_3$) ;

1.54 (m, 8H, 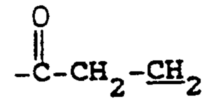);

1.78 (m, 8H, 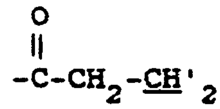);

2.24 (m, 8H, 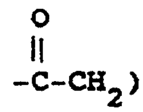);

2.59 (m, 8H, 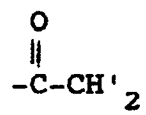);

4.23 (l, 4H, J=7, 4 Hz, CH);
5.94 (s, 2H, Ar = $H_A$);
6.75 (s, 2H, AR = $H_B$);
6.90 (s, 2H, Ar = $H'_B$);
7.36 (s, 2H, Ar = $H'_A$);

Elemental analysis for $C_{16}H_{272}O_{16}$;

| | |
|---|---|
| theoretical : | C = 78.38% ; H = 11.18% |
| found : | C = 78.42% ; H = 11.28% |

Examples 2-3

By operating as in Example 1, but using lauroyl chloride and stearoyl chloride respectively, there were obtained — 3.5. 10.12.17.19.24.26 — octadodecanoyloxy-r-1, c-8, c-15, c-22-tetramethyl-$[1_4]$metacyclophane, in 60% yield after purification through a silica gel column with 7/3 $CH_2Cl_2$/toluene as the eluent.
Mass ($DCI^+$) ; $MH^+$ = 2001
NMR ($CDCl_3$) : 0.88 ppm [t, 24H, J=6, 6Hz, $(CH_2)_n$-$CH_3$] ;

2.24 (m 8H, 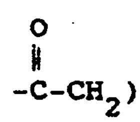);

2.59 (m 8H, 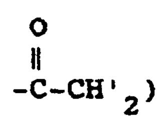);

4.22 (q, 4H, J=7, 2Hz, CH),
5.94 (s, 2H, Ar-$H_A$),
1.28 [bs, 128H, $(CH_2)_8$] ;
1.44 (d, 12H, J=7, 2Hz, CH-$CH_3$) ;

O
||
1.53 (m, 8H, - C - CH - CH)

4.22 (9, 4H, J=7, 2Hz, CH) ;
5.94 (s, 2H, Ar-$H_A$) ;
6.75 (s, 2H, AR-$H_B$) ;
6.91 (s, 2H, Ar-$H'_B$) ;
7.36 (s, 2H, Ar-$H'_A$).

Elemental analysis for $C_{128}H_{208}O_{16}$ :

theoretical : C = 76.75% ; H = 10.47%
found : C = 76.44% H = 10.52% ; and

— 3.5.10.12.17.19.24.26 — octadecanoyloxy-r-1, c-8, c-15,c-22-tetramethyl-[$l_4$]metacyclophane, with 62% yield after double purification through a silica gel column with 7/3 $CH_2Cl_2$/hexane and 6/4 $CH_2Cl_2$/hexane as the eluents.
Mass ($DCl^+$) ; $MH^+$ = 2673
NMR($CDCl_3$) :0.87 ppm [t, 24H, J=6, 7Hz, $(CH_2)_n\underline{CH_3}$]
1.28 [bs, $224H_p(CH_2)_{14}$]
1.43 (D, 12H, J=7, 5Hz, CH-$\underline{CH_3}$) ;

O
||
1.51 (m, 8H, - C-$CH_2$-$\underline{CH_2}$);

O
||
1.75 (m, 8H, - C=$CH_2$-$\underline{CH'}_2$);

O
||
2.22 (m, 8H, - C-$CH_2$);

O
||
2.57 (m, 8H, - C-$CH'_2$);

4.22 (q, 4H, J=7, 5Hz, CH) ;
5.91 (s, 2H, Ar-$H_A$) ;
6.72 (s, 2H, Ar-$H_B$) ;
6.88 (s, 2H, Ar-$H'_B$) ;
7.38 (s, 2H, Ar-$H'_A$).

Elemental analysis for $C_{176}H_{304}O_{16}$ :

theoretical : C = 78.98% ; H = 11.45%
found : C = 78.80% ; H = 11.38%

Examples 4-5 (Comparative Examples)

By operating as in Example 1, but using hexanoyl chloride and decanoyl chloride respectively two products were obtained having no columnar mesophases, namely : 3.5.10.12.17.19.24.26-octahexanoyloxy-r-1, c-8, c-15, c-22-tetramethyl-$l_4$]metacyclophane, in a yield of 68%.

(elemental analysis for $C_{80}H_{112}O_{16}$ :

theoretical : C = 72.26% ; H = 8.49%
found : C = 72.75% ; H = 8.43%
Mass ($DCI^+$) : $MH^+$ = 1329) ; and

3.5.10.12.17.19.24.26-octadecanoyloxy-r-1, c-8, c-15, c-22-tetramethyl-[$l_4$]metacyclophane, with yield of 62%.
Elemental analysis for $C_{112}H_{176}O_{16}$ ;

theoretical : C = 75.63% ; H = 9.97
found : C = 75.67% ; H = 10.05%
Mass ($DCI^+$) : $MH^+$ = 1777.

Example 6

By operating as in Example 1, using tetradecanoyl chloride, there was obtained 3.5.10.12.17.19.24.26-octatetradecanoyloxy-r-1, c-8, c-15, c-22-tetramethyl-[$l_4$]metacyclophane, in a yield of 60%.

Elemental anaysis for $C_{144}H_{240}O_{16}$ :

theoretical : C = 77.65% : H = 10.86%
found : C = 77.57% : H = 11.01%
Mass ($DCI^+$) : $MH^+$ = 2225.

In the following Table 1, there are shown the transition temperatures and enthalpies of the products disclosed in Examples 1-5.

## Table 1

**Transition Temperature (°C), Enthalpies (kJ/mol. in brackets), of Octa-alkanoyl-Tetramers**

$$R' = -\overset{\overset{\displaystyle O}{\|}}{C}-C_nH_{2n+1}$$

| n | Yields | K | | C | | I |
|---|---|---|---|---|---|---|
| 5 | 68% | . | | | 160.3 | |
| | | | | | (41.8) | |
| 9 | 62 | . | | | 40.3 | |
| | | | | | (27.2) | |
| 11 | 60% | . | 23.0 | . | 28.1 | |
| | | | (12.5) | | (27.3) | |
| 13 | 60% | . | 28.9 | . | 37.0 | |
| | | | (15.3) | | (1.0) | |
| 15 | 59% | . | 40.0 | . | 45.5 | |
| | | | (53.0) | | (48.2) | |
| 17 | 62% | . | 49.0 | . | 55.0 | |
| | | | (31.9) | | (105.6) | |

I = Isotropic phase

C = Tridimensional columnar mesophase

K = Crystalline phase

(.) = Observed phase.

Example 7

Preparation of 3,5,10,12,17,19,24,26-octa-p-dodecyloxybenzoyloxy-r-1, c-8, c-15, c-22-tetramethyl-[$l_4$]metacyclophane.

0.545 g (1 mM) of the tetramer of formula (V) (R=$CH_3$), and 5.2 g (16 mM) of p-dodecyloxybenzoyl chloride were heated for 8 hours at about 180° with stirring. After reaction, the solid residue was dissolved in $CH_2Cl_2$ and the thus-obtained solution was mixed and stirred with NaOH (0.1 N), then washed with water to a neutral pH and finally dried throughly over sodium sulphate. The residue after evaporation was chromatographed twice over silica gel with $CH_2Cl_2$ as the first eluent, and $CHCl_3$ as the second eluent. 1.17 g of pure product were obtained, yield 41%.

Mass (DCI$^+$) ; MH+= 2849

NMR($CDCL_3$) :

0.87 ppm [t, 24H, J=6, 6Hz, $(CH_2)_n$-$CH_3$] ;
1.26 [bs, 128H, $(CH_2)$8] ;
1.44 [m, 16H, O-CH-$CH_2$-$CH_2$) ;
1.58 (d, 12H, J=6, 8Hz, CH-$CH_3$) ;
1.79 (m, 16H, O-CH-$CH_2$) ;
3.93 (m, 8H, O-$CH_2$) ;
4.01 (t, 8H, J=6, 1Hz, O-$CH'_2$) ;
4.61 (q, 4H, J=6, 8Hz, CH) ;
6.38 (s, 2H, Ar-$H_A$) ;
6.73 (d, 8H, J=8, 7Hz, Ar-$H_1$) ;
6.83 (d, 8H, J=8, 7Hz, Ar-$H'_1$) ;
7.06 (s, 2H, Ar-$H_B$) ;
7.20 (s, 2H, Ar-$H'_B$) ;
7.60 (s, 2H, Ar-$H'_A$) ;
7.72 (d, 8H, J=8, 7Hz, Ar-$H_2$) ;
7.76 (d, 8H, J=8, 7Hz, Ar-$H'_2$) ;

Elemental analysis for $C_{84}H_{256}O_{24}$ :

theoretical : C = 77.49% ; H = 9.05%.

Example 8

By operating as in Example 7, using p-hexadecyloxybenzoylchloride, there was obtained, after purification through a silica gel column with 85/15 $CH_2Cl_2$/hexane as the eluent, the compound 2,3,10,12,17,19,24,26-octa-p-hexadecyloxybenzoyloxy-r-1, c-8, c-15, c-22-tetramethyl-$l_4$]metacyclophane in a yield of 44%.

Elemental analysis for $C_{216}H_{320}O_{24}$ :

| | |
|---|---|
| theoretical : | C = 78.59% ; H = 9.77% |
| found : | C = 78.67% ; H = 9.77% |
| Mass (DCI$^+$) : | MH$^+$ = 3297 |

## Table 2

Transition temperatures (°C), Enthalpy (KJ/mol in parentheses) of octa-p-alkyloxybenzoyloxy-tetramers:

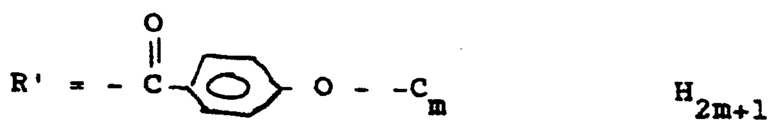

| m | Yield | K | C | | I |
|---|---|---|---|---|---|
| 12 | 41% | . | 3.0 | . | 111.0 |
| | | | (32.1) | | (36.3) |
| 16 | 44% | . | 43.0 | | 106.5 |
| | | | (124.0) | | (53.8) |

## Claims

1. Macrocylic tetramers, having stable columnar, tridimensional mesophases, of the formula :

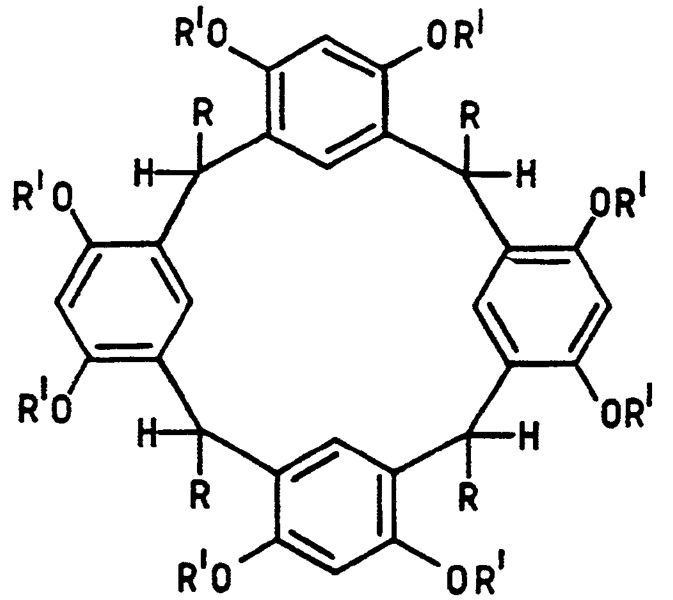

(I)

wherein

R is a $C_1$-$C_3$ alkyl group ; and, the groups R' are the same or different and each is a group of the formula:

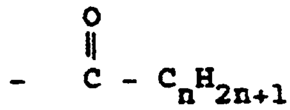 (II)

(in which n is an integer of 10 or more),

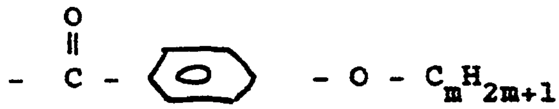 (III)

(in which m is an integer of 11 or more), or

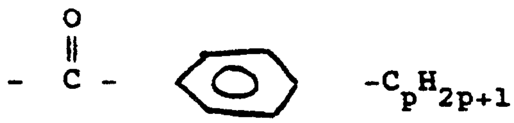 (IV)

(in which p is an integer of 11 or more).

2. A tetramer according to claim 1, wherein n is an integer of from 11 to 25, and m and p are integers of from 11 to 25.

3. A tetramer according to claim 1 or 2, wherein the groups R′ are the same.

4. A tetramer according to any one of claims 1-3 having a melting point of from 0°C to 150°C.

5. A memory device, an opto-electronic display or a non-linear optical device having as a component a macrocyclic tetramer as defined in any one of claims 1-4.

**Patentansprüche**

1. Mäkrocyclische Tetramere mit stabilen, dreidimensionalen säulenförmigen Mesophasen der Formel

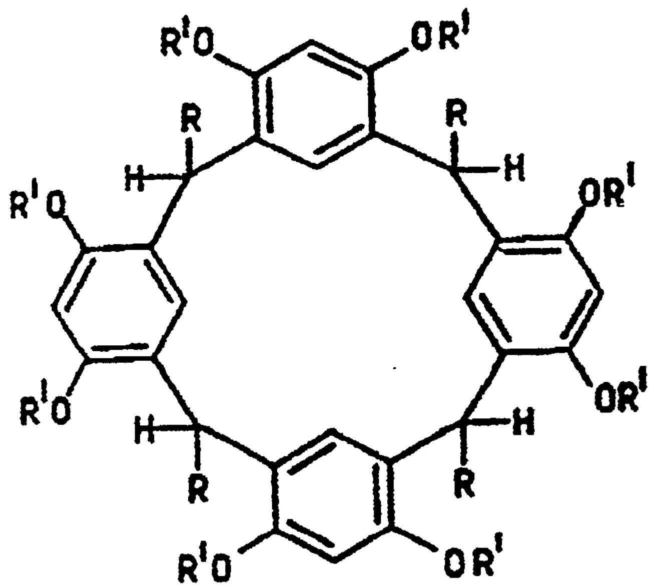 (I)

worin

R ein $C_1$-$C_3$-Alkylrest ist, und die Reste R′, die gleich oder verschieden sind, jeweils einen Rest der Formel

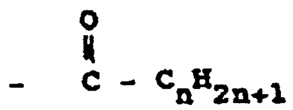 (II)

(in welcher n eine ganze Zahl von 10 oder mehr ist),

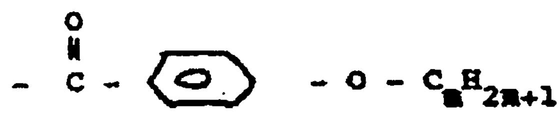 (III)

(in welcher m eine ganze Zahl von 11 oder mehr ist) oder

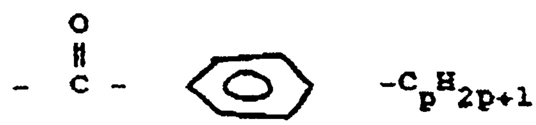 (IV)

(in welcher p eine ganze Zahl von 11 oder mehr ist), bedeuten.

2. Tetramer gemäß Anspruch 1, in dem n eine ganze Zahl zwischen 11 und 25 ist, und m und p ganze Zahlen zwischen 11 und 25 bedeuten.

3. Tetramer gemäß Anspruch 1 oder 2, in dem die Reste R′ gleich sind.

4. Tetramer gemäß irgendeinem der Ansprüche 1 bis 3, das einen Schmelzpunkt zwischen 0°C und 150°C besitzt.

5. Speichervorrichtung, optoelektronisches Display oder nicht-lineare optische Vorrichtung, die als eine Komponente ein makrocyclisches Tetramer gemäß irgendeinem der Ansprüche 1 bis 4 enthalten.

## Revendications

1. Des trétamères macrocycliques, ayant des mésophases stables tridimensionnelles en colonnes, représentés par la formule :

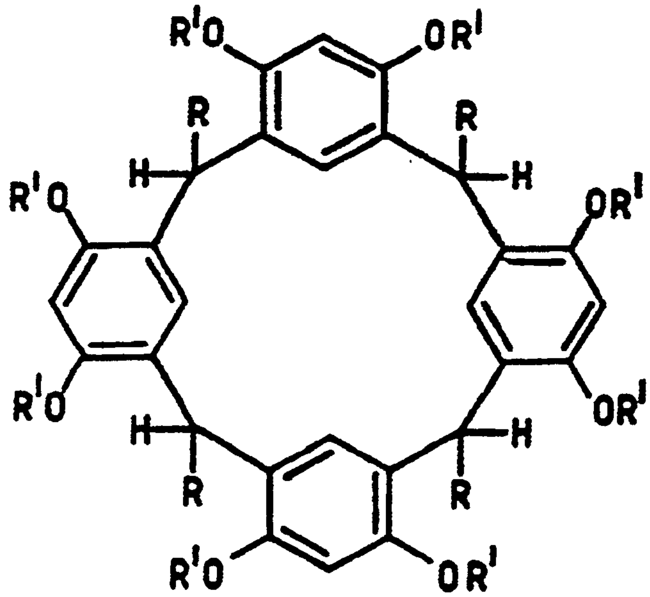

(I)

dans laquelle

R est un groupe alkyle en $C_{1-3}$ ; et les groupes R′ sont identiques ou différents et sont chacun un groupe de formule :

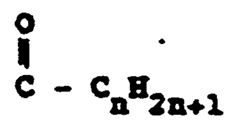

(II)

(dans laquelle n est un entier de 10 ou plus),

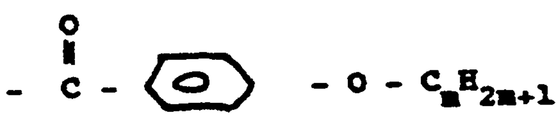

(III)

(dans laquelle m est un entier de 11 ou plus), ou

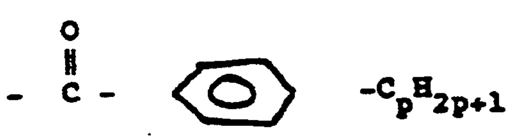

(IV)

(dans laquelle p est un entier de 11 ou plus).

2. Un tétramère suivant la revendication 1, dans lequel n est un entier de 11 à 25, et m et p sont des entiers de 11 à 25.

3. Un tétramère suivant la revendication 1 ou la revendication 2, dans lequel les groupes R′ sont identiques.

4. Un tétramère suivant l'une quelconque des revendications 1 à 3, ayant un point de fusion de 0°à 150°C.

5. Un dispositif de mémoire, un affichage optoélectronique ou un dispositif optique non linéaire ayant en tant que composant un tétramère suivant l'une quelconque des revendications 1 à 4.

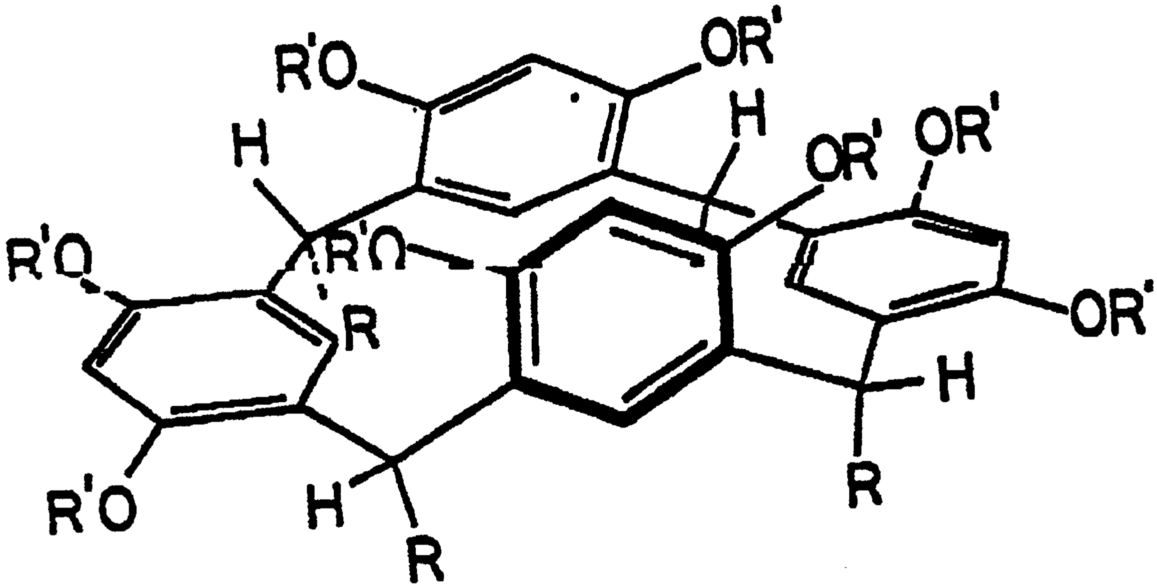

Fig. 1

Fig. 2